# EUROPÄISCHE PATENTSCHRIFT

(11) **EP 0 423 525 B1**
(45) Veröffentlichungstag und Bekanntmachung des Hinweises auf die Patenterteilung: **23.06.1993**
(21) Anmeldenummer: 90118754.2
(22) Anmeldetag: 29.09.1990
(51) Int. Cl.: C07C 31/26, C07C 29/141, C07H 3/04

(54) **Verfahren zur Herstellung von epimerenfreien Zuckeralkoholen aus der Gruppe von Xylitol, Sorbitol (D-Glucitol), 4-O-beta-D-Galactopyranosyl-D-glucitol und 4-O-alpha-D-Glucopyranosyl-D-sorbitol**
Process for the preparation of epimer-free sugaralcohols from the group of xylitol, sorbitol (D-glucitol), 4-0-beta-D-galactopyranosyl-D-glucitol and 4-0-alpha-D-glycopyranosyl-D-sorbitol
Procédé de préparation d'alcools exempts d'épimères, dérivés de sucres, choisis parmi le xylitol, le sorbitol (D-glucitol), 4-0-bêta-D-galactopyranosyl-D-glucitol et 4-0-alpha-D-glycopyranosyl-D-sorbitol

(30) Priorität: 14.10.1989 DE 3934458; 14.10.1989 DE 3934457; 25.11.1989 DE 3939058; 13.01.1990 DE 4000839
(43) Veröffentlichungstag der Anmeldung: 24.04.1991
(73) Patentinhaber: BAYER AG, 51368 Leverkusen (DE)
(72) Erfinder: Darsow, Gerhard, Dr., D-4150 Krefeld (DE)

(56) Entgegenhaltungen:
- EP-A- 0 152 779
- US-A- 4 322 569

## Beschreibung

Die Erfindung betrifft ein Verfahren zur Herstellung der als Titelverbindungen genannten Zuckeralkohole in epimerenfreier Form aus den korrespondierenden Zuckern Xylose, α-D-Glucose, 4-O-β-D-Galactopyranosyl-α-D-sorbitol bzw. 4-O-α-D-Glucopyranosyl-α-D-glucopyranose durch kontinuierliche katalytische Hydrierung mit Wasserstoff. Der Reaktionsverlauf läßt sich durch die folgenden Reaktionsschemata veranschaulichen: 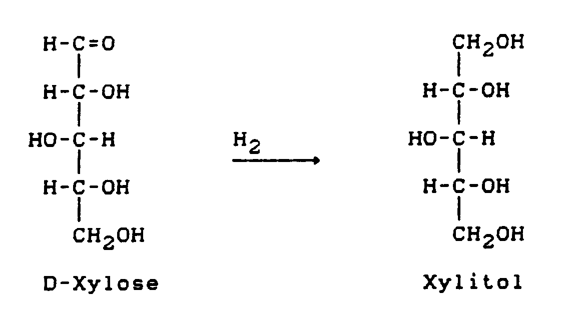 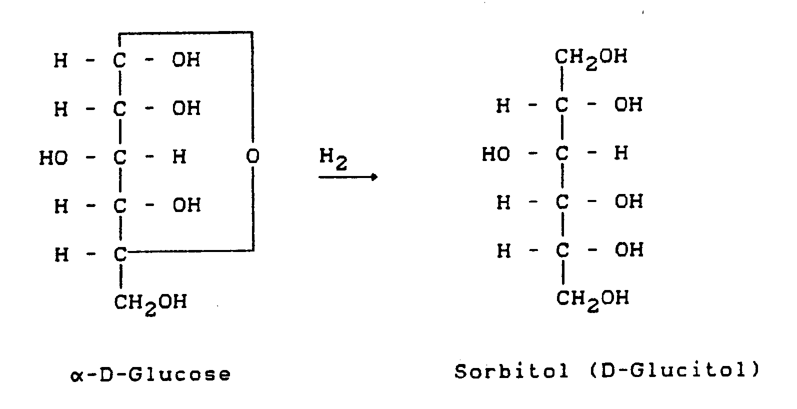 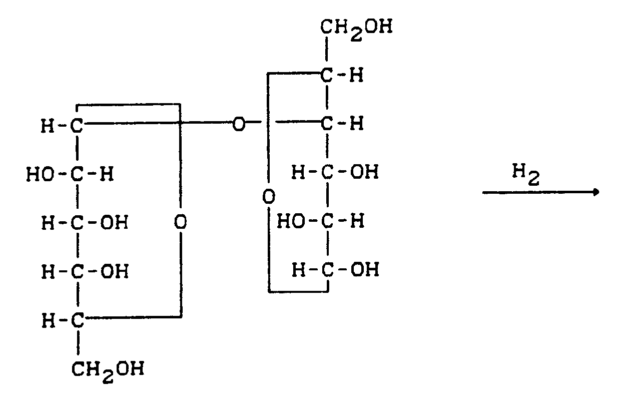
4-O-β-D-Galactopyranosyl-α-D-glucopyranose (Lactose, Milchzucker) 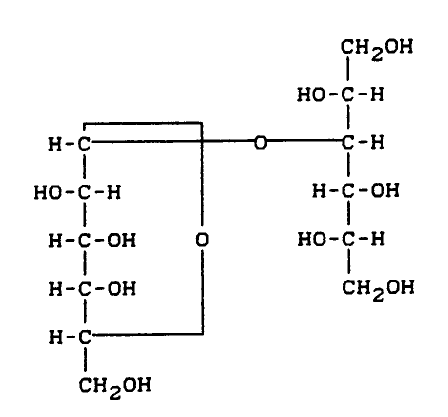
4-O-β-D-Galactopyranosyl-D-glucitol (Lactitol) 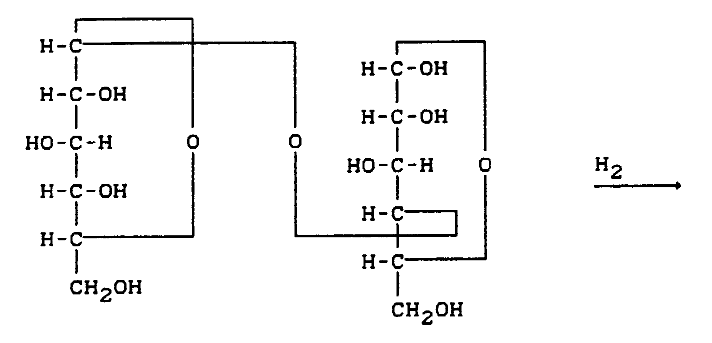
4-O-α-D-Glucopyranosyl-α-D-glucopyranose (Maltose) 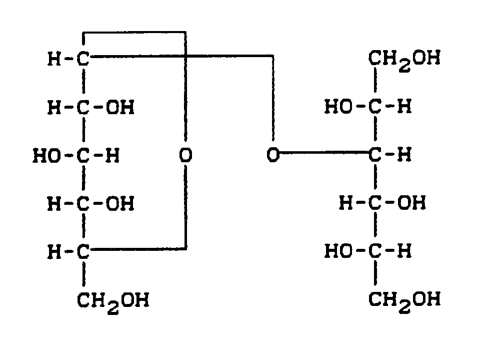
4-O-α-D-Glucopyranosyl-D-sorbitol (Maltitol)

Die vier erfindungsgemäß in epimerenfreier Form herstellbaren Zuckeralkohole sind bekannt und können wie folgt beschrieben werden:

Die Süßkraft von Xylitol erreicht etwa 80-100 % der Süßkraft von Saccharose. Zur Erhöhung der Süßkraft kann die wäßrige Lösung mit künstlichen Süßstoffen, beispielsweise Cyclohexylsulfamat oder Phenylalanin-asparaginsäure-methylester, versetzt und durch gemeinsame Vakuumkristallisation in kristalliner Form gewonnen werden. Man kann aber auch die künstlichen Süßstoffe in fester Form mit dem Xylitol-Kristallisat vermischen. Xylitol kann auch in flüssiger oder fester Form mit anderen süß schmeckenden Kohlenhydraten, beispielsweise Maltitol, Lactitol, Sorbitol und anderen vermischt werden.

Wegen seines angenehm süßen Geschmacks eignet sich Xylitol als Zuckeraustauschstoff in der Diabetiker-Diät und als nicht kariogenes Süßungsmittel in Süßwaren und oralen Pharmazeutika. Für Diabetiker-Lebensmittel ist Xylitol durch die Diätverordnung (Bundesgesetzblatt I 1982, Seite 71) ohne Mengenbegrenzung zugelassen.

Reines Xylitol ist besonders gut zur Verarbeitung in Süßwaren, wie Bonbons und Kaugummi, geeignet (Swiss Dent. I (7/8) 1980, Seite 25 bis 27). Auch Produkte zur Behandlung des Mund- und Rachenraumes, wie Zahnpasten, rachendesinfizierende Tabletten, Hustenbonbons, werden zunehmend mit Xylitol gesüßt (Swiss Dent. III (7/8) 1982, Seite 25 bis 30).

Sorbitol wird im menschlichen Körper nur in untergeordnetem Maße resorbiert und nur in diesem Anteil abgebaut. Daher ist Sorbitol als Zuckerersatzstoff für Diabetiker und als kalorienarmer Süßstoff geeignet. Ferner ist es weniger kariogen als Glucose oder andere Zucker.

Die Süßkraft von Sorbitol erreicht etwa 50-60 % der Süßkraft von Saccharose. Zur Erhöhung der Süßkraft kann die wäßrige Lösung mit künstlichen Süßstoffen, beispielsweise Cyclohexylsulfamat oder Phenylalanin-asparaginsäure-methylester, versetzt und durch gemeinsame Vakuumkristallisation in kristalliner Form gewonnen werden. Man kann aber auch die künstlichen Süßstoffe in fester Form mit dem Sorbitol-Kristallisat vermischen. Sorbitol kann auch in flüssiger oder fester Form mit anderen süß schmeckenden Kohlenhydraten, beispielsweise Maltitol, Lactitol, Xylitol und anderen vermischt werden.

Toxische Effekte konnten auch in Langzeitstudien nicht festgestellt werden (Ullmanns Encyklopädie der technischen Chemie, Band 24, Weinheim 1983, S. 774), so daß sich vielseitige Anwendungsmöglichkeiten im Lebensmittelbereich, bei der Herstellung von Diabetikerprodukten sowie von zuckerfreien Süßigkeiten und Nahrungsmitteln mit geringem Nährwert ergeben.

Zur Herstellung von Xylitol oder Sorbitol wird vielfach ein diskontinuierliches Verfahren (Batch-Prozeß) angewandt, bei dem ein pulverförmiger Nickel-Katalysator in einem Suspensionsverfahren zum Einsatz kommt.

Lactitol wird im menschlichen Körper nicht als Kohlenhydrat abgebaut und im Dünndarm weder hydrolysiert noch absorbiert. Daher ist Lactitol als Zuckerersatzstoff für Diabetiker geeignet. Ferner ist es weniger kariogen als Saccharose.

Die Süßkraft von Lactitol erreicht etwa 40 % der Süßkraft von Saccharose. Zur Erhöhung der Süßkraft kann die wäßrige Lösung mit künstlichen Süßstoffen, beispielsweise Cyclohexylsulfamat oder Phenylalanin-asparaginsäure-methylester, versetzt und durch gemeinsame Vakuumkristallisation in kristalliner Form gewonnen werden. Man kann aber auch die künstlichen Süßstoffe in fester Form mit dem Lactitol-Kristallisat vermischen. Lactitol kann auch in flüssiger oder fester Form mit anderen süß schmeckenden Kohlenhydraten, beispielsweise Fructose, Sorbitol, Xylitol und anderen vermischt werden.

Toxische Effekte konnten auch in Langzeitstudien nicht festgestellt werden (Ullmanns Encyklopädie der technischen Chemie, Band 24, Weinheim 1983, S. 779), so daß sich vielseitige Anwendungsmöglichkeiten im Lebensmittelbereich, bei der Herstellung von Diabetikerprodukten sowie von zuckerfreien Süßigkeiten und Nahrungsmitteln mit geringem Nährwert ergeben.

Zur Herstellung des bisher in der Natur nicht nachgewiesenen Lactitols ist aus EP 39 981 ein diskontinuierliches Verfahren (Batch-Prozeß) bekannt, bei dem ein pulverförmiger Nickel-Katalysator in einem Suspensionsverfahren zum Einsatz kam.

Maltitol wird im menschlichen Körper durch amylolytische Enzyme nur schwer abgebaut. Daher ist Maltitol als Zuckerersatzstoff für kalorienreduzierte Diät und für Diabetiker geeignet (Ullmann's Encyklopädie der technischen Chemie, Band 24, Weinheim 1983, S. 771).

Die Süßkraft von Maltitol erreicht die Süßkraft von Saccharose. Maltitol kann in flüssiger Form mit anderen süß schmeckenden Kohlenhydraten, beispielsweise Fructose, Sorbitol, Xylitol und anderen vermischt werden. Besonders der Einsatz in der Getränkeindustrie empfiehlt sich wegen seiner hohen Süßkraft und seiner geringen Neigung zur Kristallisation selbst in hohen Konzentrationen.

Zur Herstellung des bisher in der Natur nicht nachgewiesenen Maltitols ist aus US 3.741 776 ein diskontinuierliches Verfahren (Batch-Prozeß) bekannt, bei dem ein pulverförmiger Nickel-Katalysator in einem Suspensionsverfahren zum Einsatz kam.

Diskontinuierliche Verfahren haben den Nachteil, daß ihre Kapazität, relativ zum Reaktionsvolumen, sehr klein ist und somit ein Bedarf nach großen Reaktionsapparaturen und Lagertanks besteht. Der Energieverbrauch ist unökonomisch, und der Personalbedarf ist verhältnismäßig hoch.

Kontinuierliche Pulverkatalysator-Verfahren, die mit mehreren in Kaskade geschalteten Hydrierreaktoren arbeiten, vermeiden einen Teil der genannten Nachteile. Es bleibt jedoch der umständliche Weg, den pulverförmigen Katalysator gezielt zu aktivieren, umzupumpen und quantitativ vom Reaktionsprodukt abzufiltrieren. Die Katalysatorschlammpumpen unterliegen einer hohen mechanischen Beanspruchung. Die quantitative Entfernung des pulverförmigen Katalysators ist aufwendig (Grob- und Feinfiltrationsapparate in Wechselausführung). Ferner ist die Gefahr groß, daß der Katalysator durch die zusätzlichen Operationen verhältnismäßig schnell seine Aktivität verliert (hoher Katalysatorverbrauch). Es ist daher wünschenswert, die Reaktion über fest angeordneten Katalysatoren ablaufen zu lassen, welche eine hohe spezifische Aktivität besitzen sollen, die auch über einen längeren Zeitraum von 1 bis mehreren Jahren nicht nachlassen soll, weil häufige Katalysatorwechsel auch bei Festbettreaktionen aufwendig sind.

Auch bei fest angeordneten Katalysatoren ist es üblich, mehrere Reaktoren hintereinander zu schalten, wodurch sich mehrere in Serie geschaltete Reaktionszonen ergeben (DE-OS 32 14 432).

Zum Einsatz kommen Nickelkatalysatoren auf Trägermaterial (SiO₂/Al₂O₃) mit extrem hohen aktiven Oberflächen von 140-180 m² /g, so daß die Katalysatoren derart aktiv sind, daß sie durch zusätzliche chemische Behandlungsmethoden stabilisiert werden müssen, beispielsweise durch Sauerstoffbegasung zur Bildung monomolekularer Sauerstoffschichten auf der Katalysatoroberfläche (DE-OS 31 10 493). Die desaktivierende Stabilisierung des Katalysators macht aber dann so hohe Reaktionstemperaturen bei der Hydrierung von Zuckern erforderlich (130-180° C), daß unkontrollierbare Nebenreaktionen möglich werden, wie Verfärbung durch Karamelisierung und hydrierende Crackung (Hydrogenolyse) der Saccharidalkohole bis zur Bildung von Methanol und sogar Methan. Außerdem gehen bei dieser Reaktionsweise stets Schwermetalle wie Nickel, Eisen oder Kobalt in ionischer oder kolloider Form in Lösung, was einmal eine nachträgliche Aktivkohlebehandlung des hydrierten Produktes und zum anderen eine Entionisierung durch Ionenaustauscher erforderlich macht.

Da die bekannten Hydrierverfahren mit auf pH-Werte von 7-13 eingestellten Zuckerlösungen arbeiten, sind den Ausgangslösungen Alkalien oder Erdalkalien beizugeben, die ebenfalls vom Endprodukt wieder umständlich entfernt werden müssen (DE-OS 31 10 493; DE-OS 32 14 432).

Weiterhin ist zu erwarten, daß unter den hydrierenden Bedingungen eine merkliche Epimerisierung eintritt, so daß beispielsweise aus der D-Xylose neben Xylitol auch Lyxitol (Arabinitol und Ribitol) erhalten werden; aus α-D-Glucose wäre neben Sorbitol auch Mannitol zu erwarten.

Ferner ist der Effekt der Spaltung der Kohlenstoffkette von Zuckern bei der katalytischen Hydrierung an Raney-Nickel bekannt; DE-OS 27 56 270 beschreibt diesen Effekt an einem Zuckergemisch, wie es aus der Selbstkondensation von Formaldehyd hervorgeht, wobei im Rahmen der dortigen Ausführungsbeispiele eine deutliche Verschiebung von höheren C-Kettenzahlen zu niedrigeren beobachtet wird. Gemäß DE-OS 28 31 659 wird eine solche Spaltung an Edelmetallkatalysatoren der 8. Nebengruppe nicht beobachtet.

Aus EP 152 779 ist ein Verfahren zur Hydrierung von α-D-Glucopyranosido-1,6-fructose an fest angeordneten Katalysatoren bekannt. Hierbei wird jedoch ein Gemisch zweier Reduktionsprodukte, nämlich von α-D-Glucopyranosido-1,6-mannit und α-D-Glucopyranosido-1,6-sorbit erhalten, deren Verhältnis etwa 1:1 beträgt.

Es war daher überraschend, daß im vorliegenden Verfahren die Zucker nicht nur nahezu vollständig umgesetzt werden, sondern unter Vermeidung von Epimerisierung und C-Kettenspaltung sowie unter Vermeidung der Bildung höhermolekularer Komponenten durch Kondensationsreaktionen unter Etherbildung nur ein Zuckeralkohol, wie weiter vorn in den Reaktionsschemata angegeben, erhalten wird. Dies ist wichtig für den unmittelbaren Einsatz als Diätikum ohne weitere Reinigung.

Es wurde ein Verfahren zur Herstellung von epimerenfreien Zuckeralkoholen aus der Gruppe von Xylitol, Sorbitol (D-Glucitol), 4-O-β-D-Galactopyranosyl-D-glucitol (Lactitol) und 4-O-α-D-Glucopyranosyl-D-sorbitol (Maltitol) durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur gefunden, das dadurch gekennzeichnet ist, daß man die Hydrierung kontinuierlich bei einem Wasserstoffdruck von 100-500 bar und Temperaturen von 60-125°C im Festbettverfahren in einer Reaktionszone über als Hydrierungskatalysatoren dienenden trägerfreien Formkörpern mit einer Druckfestigkeit von mehr als 50 N, vorzugsweise 100-400 N, auf die Formkörperoberfläche und einer inneren Oberfläche von 10 bis 90 m² /g von einem oder mehreren Elementen der Eisengruppe des Periodensystems durchführt.

Als epimerenfrei im Sinne der vorliegenen Erfindung gilt ein für die Reinheit der genannten Zuckeralkohole insoweit vernachlässigbar Gehalt an Epimeren, daß diese Zuckeralkohole die handelsüblichen Spezifikationen, wie sie im Deutschen Arzneimittelbuch (DAB) sowie in der United States Pharmacopeia (USP) und im Food Chemicals Codex (FCC) angegeben sind, ohne weitere Reinigungsgänge erfüllen.

Die Zuckeralkohole werden im erfindungsgemäßen Verfahren in nahezuquantitativer Ausbeute erhalten. Dies ist von besonderer Bedeutung, da die Entfernung (durch Etherbildung entstandener) höhermolekularer oder (durch Hydrogenolyse entstandener) niedermolekularer störender Verunreinigungen aus dem Reaktionsprodukt durch zusätzliche Reinigungsprozesse, wie Umkristallisation aus Lösungsmitteln, normalerweise einen beträchtlichen ökologischen Aufwand hinsichtlich deren Entsorgung erfordert. Die dem Xylitol epimeren Zuckeralkohole vom Typ des Lyxitols (Arabitinol und Ribitol) sind im Reaktionsprodukt höchstens spurenweise (Summe < 0,3 %) nachweisbar. Das zum Sorbitol diastereomere Mannitol ist im Reaktionsprodukt höchstens spurenweise (< 0,3 %) enthalten. Das zum Lactitol diastereomere 4-O-β-D-Galactopyranosyl-D-mannitol und das zum Maltitol diastereomere 4-O-α-D-Glucopyranosyl-D-mannitol sind im jeweiligen Reaktionsprodukt nicht nachweisbar.

Die erfindungsgemäß einzusetzenden Festbettkatalysatoren neigen im Gegensatz zu trägerhaltigen Katalysatoren nicht zum "Ausbluten", d.h. nicht zum Übergang von Katalysatorbestandteilen in ionischer oder kolloider Form in die Lösungsphase des Substrats, so daß das Substrat nicht durch Schwermetalle kontaminiert wird, die normalerweise ebenfalls nur mühsam, beispielsweise mit Hilfe von Ionenaustauschern, unter ökologischem Aufwand aus dem Substrat entfernt werden können. Die einzusetzenden Katalysatoren können nach ihrem Gebrauch leicht aufgearbeitet werden, da die Schwermetalle nicht umständlich von einem Trägermaterial getrennt werden müssen. Bei Polyhydroxylverbindungen war weiterhin die Neigung zu befürchten, mit Schwermetallionen komplexe Chelatverbindungen zu bilden, die nur schwierig aus Lösungen entfernt werden können.

Es ist demnach möglich, im erfindungsgemäßen Verfahren den jeweiligen Zuckeralkohol in einer Reinheit von über 99 % in der Trockenmasse herzustellen. Der Gehalt an nicht umgesetztem Zucker erreicht Werte von lediglich 0,2 % oder darunter. Da die glykosidischen Etherbindungen bei Disacchariden geschont werden, liegen die Gehalte der Monozuckeralkohole nur bei 0,3 % oder darunter. Die handelsüblichen Spezifikationen für die Zuckeralkohole, etwa nach DAB, USP oder FCC, können daher normalerweise ohne weitere Reinigungsvorgänge direkt erfüllt werden.

Als Ausgangsverbindung für das erfindungsgemäße Verfahren wird reine kristalline D-Xylose bzw. reine α-D-Glucose bzw. reine α-Lactose oder α-Lactose-Monohydrat bzw. reine Maltose (flüssig oder in ihrer β-Form auch als kristallines Monohydrat) eingesetzt. Der Einsatzstoff wird in Sauerstoff-freiem entionisierten Wasser so gelöst, daß eine 15-45 gew.-%ige, bevorzugt 35-40 gew.-%ige Lösung entsteht, deren pH-Wert 3,5-10,5 beträgt. Die Lösung der Monosaccharide wird bevorzugt auf einen pH-Wert von 3,5-8, die der Disaccharide bevorzugt auf einen pH-Wert von 5,5-10,5 eingestellt. Für alle Ausgangsstoffe ist der besonders bevorzugte pH-Bereich 6-7,5. Die als Ausgangsverbindungen benannten Zucker zeigen, gelöst in Wasser mit einem pH-Wert von 7, eine neutrale oder, bedingt durch spurenweise Bildung von Zuckersäuren, eine schwach saure Reaktion, können aber in einer dem Fachmann bekannten Weise, beispielsweise durch Zugabe von Sorbinsäure oder Zitronensäure auf den gewünschten pH-Wert eingestellt werden.

Für die erfindungsgemäße Hydrierung wird reiner Wasserstoff eingesetzt, der auf einen Druck von 100-500 bar, bevorzugt 150-300 bar, vorkomprimiert wird. Die Hydrierung erfolgt kontinuierlich im Festbettverfahren an den weiter unten beschriebenen Katalysatoren, indem man die zu hydrierende Lösung entweder im Gleichstrom von unten aufsteigend gemeinsam mit dem vorher zugemischten Wasserstoff über den in einem Hydrierreaktor angebrachten Katalysator strömen läßt (Gleichstromverfahren) oder aber, indem man die von unten aufsteigende zu hydrierende Lösung dem von oben einströmenden Wasserstoff entgegenführt (Gegenstromverfahren).

Der Hydrierreaktor kann entweder ein einzelnes Hochdruckrohr aus Stahl oder einer Stahllegierung sein, das mit dem Katalysator ganz oder teilweise gefüllt wird, wobei bei gewissen Rohrquerschnitten auch die Anwendung des Katalysators auf Horden (Drahtkörbe oder ähnliches) nützlich sein kann, oder aber ein ummanteltes Hochdruckrohrbündel, dessen Einzelrohre mit Katalysator ganz oder teilweise gefüllt werden. Weiterhin kann anstelle eines größeren Einzelrohrreaktors eine Anordnung von mehreren kleinen Einzelrohrreaktoren hintereinander in einer Kaskade betrieben werden.

Die trägerfreien Katalysatoren werden aus Metallpulvern der Elemente der Eisengruppe des Periodensystems, insbesondere Nickel, Kobalt oder ihren Gemischen oder Legierungen untereinander oder mit Eisen, besonders bevorzugt aus Nickel oder aus mindestens zu 70 % aus Nickel bestehenden Mischungen zu Formkörpern verarbeitet. Hierbei können Pulver der einzelnen Metalle oder pulverisierte Legierungen der genannten Metalle zum Einsatz kommen. Die Herstellung von Formkörpern erfolgt nach gebräuchlichen Methoden durch Verpressen der Metallpulver, beispielsweise auf Tablettier-oder Pelletiermaschinen, unter hohem Druck, wobei zur Verbesserung des Haftvermögens der Metallpartikel auch Graphit und/oder Klebstoffe in Mengen von 0,5-3 Gew.-%, bezogen auf das Gesamtgewicht der den Katalysator bildenden Bestandteile, zum Einsatz kommen können. Die Herstellung der Formkörper erfolgt in einer Sauerstoff-freien Atmosphäre, um Oberflächenoxidationen zu vermeiden. Beispiele für Formkörper sind Tabletten, Kugeln oder Granulate mit Durchmessern von 3-7 mm. Tablettierte Formkörper können weiterhin zur Vergrößerung der äußeren Oberfläche mit einer axialen Durchbohrung (hole core) versehen sein. Solche Formkörper haben makroskopisch eine glatte Oberfläche.

Die erfindungsgemäß einzusetzenden als Formkörper ausgebildeten Katalysatoren müssen in einer dem Fachmann bekannten Weise so hergestellt werden, daß die dabei entstehenden Formkörper Druckfestigkeiten oberhalb von 50 N, bevorzugt Druckfestigkeiten von 100-400 N, auf die Formkörperoberfläche erhalten. Dies ist wichtig, weil niedrigere Druckfestigkeiten zu einem Zerfall bzw. zu erosivem Abrieb der Formkörper führen, was eine unerwünschte Kontaminierung des Reaktionsproduktes mit Metallpulver bewirken würde. Die erfindungsgemäß einzusetzenden Katalysatorformkörper haben weiterhin spezifische innere Oberflächen, die bei Werten von 10-90 m² /g liegen. Die Überprüfung von hergestellten Formkörpern auf solche inneren Oberflächen und damit auf Brauchbarkeit für das erfindungsgemäße Verfahren werden nach Methoden durchgeführt, die von F.M. Nelsen und F.T. Eggertsen, Analyt. Chem. 30 (1958), 1387 bzw. von S.J. Gregg und S.W. Sing, Adsorption, Surface Area and Porosity, London 1967, Kap. 2 und 8 beschrieben wurden.

Die Hydrierung wird bei Temperaturen von 60-125°C, bevorzugt 70-115°C durchgeführt. Niedrigere Temperaturen würden hohe Verweilzeiten oder dem Verzicht auf einen weitgehend quantitativen Umsatz der Zucker ergeben. Höhere Temperaturen führen zu unkontrollierbaren Nebenreaktionen, wie Karamelisierung, Etherspaltung oder hydrierender Crackung, was zu Verfärbungen sowie zur Bildung unerwünschter Nebenprodukte führen kann.

Die stündliche Katalysatorbelastung liegt bei 25-100 g der als Ausgangsverbindungen benannten Zucker pro Liter Katalysator. Bei Einhaltung der genannten Reaktionsbedingungen sind unerwartet hohe Katalysatorstandzeiten von 16 000 Stunden und mehr zu erreichen, wobei spezifische Katalysatorverbräuche von höchstens 0,1 % oder weniger erreicht werden. Damit liegen die technischen Vorteile des erfindungsgemäßen Verfahrens außer in der durch den nahezu quantitativen Umsatz bedingten hohen Ausbeute und den ökologischen Vorteilen, die sich aus der Reinheit des hergestellten Produktes ergeben, in der kostensparenden kontinuierlichen Arbeitsweise und dem extrem niedrigen Katalysatorverbrauch.

Die den Reaktor verlassende wäßrige Lösung des Zuckeralkohols kann nach der Entspannung, bei welcher man den überschüssigen Wasserstoff abfangen und nach erfolgter Komprimierung und Ergänzung durch weiteren Wasserstoff erneut zum Einsatz bringen kann, bereits direkt als Zuckeraustauschstoff in flüssiger Form Verwendung finden.

Das Wasser dieser Lösung läßt sich aber auch auf verschiedene Weise, beispielsweise über Sprühtrockner, Trockenwalzen oder über Gefriertrocknung, entfernen. Als günstig hat sich erwiesen, die erhaltene farblose und glasklare Lösung des Zuckeralkohols in einem Fallfilmverdampfer oder einem ähnlich arbeitenden Gerät auf einen Zuckeralkoholgehalt von etwa 70-80 Gew.-% aufzukonzentrieren und anschließend nach weiterer Eindampfung in einem Vakuum-Kristallisationsgerät unter Kühlung zur teilweisen oder vollständigen Kristallisation zu bringen. Das Kristallisat läßt sich durch einen nachgeschalteten Mahlungsprozeß und evtl. Siebung auf eine einheitliche Korngröße bringen. Das erhaltene Produkt ist rieselfähig.

Xylitol hat einen Schmelzpunkt von 93-94°C. Beim Sorbitol können in Abhängigkeit von den Kristallisationsbedingungen verschiedene kristalline Modifikationen hergestellt werden, von denen die γ-Form mit einem Schmelzpunkt von 101°C die stabilste ist.

In Abhängigkeit von den Kristallisationsumständen kann Lactitol entweder als Dihydrat mit einem Schmelzpunkt von 76-78°C oder als Monohydrat mit einem Schmelzpunkt von 121-123° hergestellt werden. Die Löslichkeit beider Hydrate in Wasser ist unterschiedlich; das Monohydrat ist weniger löslich als das Dihydrat. Die Hydrate sind nicht hygroskopisch und zeigen daher technologische Vorteile gegenüber anderen Polyolen. Wasserfreies Lactitol kann aus Lösungen in absolutem Ethanol in kristalliner Form gewonnen werden. Es schmilzt bei 146°C und ist hygroskopisch.

Wasserfreies Maltitol kann aus Lösungen in absolutem Ethanol als amorphes Pulver gewonnen werden. Es schmilzt bei 146-148°C und ist hygroskopisch (Helv. Chim. Acta 20, (1937), 86-90).

Alle erfindungsgemäß hergestellten Zuckeralkohole haben einen Gehalt an Katalysatorbestandteilen von unter 1 ppm.

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m² /g aufwies. Durch dieses Rohr wurden gemeinsam mit der dreifach molaren Menge von unter einem Druck von 300 bar stehendem, hochreinem Wasserstoff stündlich 250 ml einer 40 %igen Lösung von D-Xylose in entionisiertem Sauerstoff-freiem Trinkwasser mit einem pH-Wert von 7,0 kontinuierlich gepumpt und zwar von unten nach oben aufsteigend.

Wäßrige Lösung und Wasserstoff wurden vorab gemeinsam durch einen Wärmeaustauscher geführt und so hoch erhitzt, daß sie in das Hochdruckrohr mit einer Temperatur von 95°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer D-Xylose-Lösung in den Vorwärmer und von da erneut in das Hochdruckrohr gepumpt wurde.

Die farblose und klare wäßrige Lösung wurde entspannt und in einem Fallfilmverdampfer auf einen Zuckeralkoholgehalt von ca. 70 % aufkonzentriert und anschließend nach weiterer Eindampfung in einem Vakuumkristaller unter Kühlung zur Kristallisation gebracht. Es wurde ein weißes, leicht hygroskopisches, geruchloses Festprodukt erhalten, das zu einem feinkristallinen Pulver vermahlen wurde. Das gebildete Xylitol war ansonsten hochrein und zeigte in der stabilen rhombischen Kristallform einen Schmelzpunkt von 93-94°C. Der Gehalt an nicht hydrierter D-Xylose lag bei ≦0,1 %. Der Ni-Gehalt lag bei <1 ppm. Der Katalysator war auch nach einer Laufzeit von 5600 Stunden unverändert wirksam.

### Beispiel 2

Durch ein Hochdruckrohr wie in Beispiel 1 wurde bei einer Temperatur von 105°C und einem Wasserstoffdruck von 150 bar der Wasserstoff im umgekehrten Reaktionsfluß wie in Beispiel 1 der aufsteigenden Lösung von D-Xylose entgegengeführt, wobei stündlich eine gleichgroße Menge 40 %iger wäßriger Lösung von D-Xylose hydriert wurde, die einen pH-Wert von 6,5 aufwies. Der Katalysator war durch Tablettierung von Nickelpulver hergestellt worden. Die Tabletten hatten bei einer Zylinderhöhe von 5 mm und einem Durchmesser von 5 mm eine Druckfestigkeit von 149 N auf die Zylindermantelfläche und eine innere Oberfläche von 62 m² /g.

Nach einer Laufzeit von 2800 Stunden mit unverminderter Wirksamkeit lag der Umsatz der D-Xylose bei ≧99,9 %. Der Gehalt an nicht hydrierter D-Xylose im auskristallisierten Xylitol, das einen Reinheitsgrad von ≧99,5 % aufwies, betrug ≦0,1 %. Der Ni-Gehalt lag bei <1 ppm.

### Beispiel 3

In einem Hochdruckrohr wie in Beispiel 1 wurde bei einer Temperatur von 115°C und einem Wasserstoffdruck von 300 bar in gleicher Weise wie in Beispiel 1 stündlich eine gleichgroße Menge einer 40 %igen wäßrigen Lösung von D-Xylose hydriert, die einen pH-Wert von 7,5 aufwies. Der Katalysator war durch Tablettierung einer pulverisierten Nickel-Eisenlegierung gewonnen worden. Die Legierung enthielt einen Eisenanteil in Nickel von 15 %. Die Tabletten hatten bei einer Zylinderhöhe von 5 mm und einem Durchmesser von 5 mm eine Druckfestigkeit von 137 N auf die Zylindermantelfläche und eine innere Oberfläche von 83 m² /g. Das in einem Vakuumkristaller unter Animpfung mit Impfkristallen gewonnene kristalline Xylitol hatte einen Reinheitsgrad von ≧99,5 %. Der Gehalt an nicht umgesetzter D-Xylose lag bei 0,1 %. Ni- und Fe-Gehalt lagen zusammen bei <1 ppm. Der Katalysator war nach einer Laufzeit von 5400 Stunden noch unverändert wirksam.

### Beispiel 4

In einem Hochdruckrohr wie in Beispiel 1 wurde bei einer Temperatur von 110°C und einem Wasserstoffdruck von 300 bar in gleicher Weise wie in Beispiel 1 stündlich 150 ml einer 40 %igen wäßrigen Lösung von D-Xylose hydriert, die einen pH-Wert von 6,5 aufwies. Der Katalysator war durch Tablettierung von Kobaltpulver gewonnen worden. Die Tabletten hatten bei einer Zylinderhöhe von 5 mm und einem Durchmesser von 5 mm eine Druckfestigkeit von 225 N auf die Zylindermantelfläche und eine innere Oberfläche von 19 m²/g. Das in einem Vakuum-Drehrohr gewonnene Xylitol zeigte einen Gehalt an nicht umgesetzter D-Xylose von ≦0,2 %. Der Co-Gehalt lag bei <1 ppm. Der Katalysator war nach einer Laufzeit von 1000 Stunden noch unvermindert wirksam.

### Beispiel 5 (Vergleichsbeispiel)

Durch ein Hochdruckrohr wie in Beispiel 1 wurde bei einer Temperatur von 110°C und einem Wasserstoffdruck von 300 bar in gleicher Weise wie in Beispiel 1 in der gleichen Zeit eine ebenso große Menge einer 40 %igen wäßrigen Lösung von D-Xylose hydriert, die einen pH-Wert von 6,5 aufwies. Der Katalysator war durch Auftrag einer wäßrigen Nickelsalz-Lösung auf einem inerten kugelförmigen Al₂O₃-Träger (Kugeldurchmesser: 5 mm) und nachfolgende Überführung des Nickels in den metallischen Zustand durch Reduzierung im Wasserstoffstrom hergestellt worden. Der Nickelgehalt des Katalysators lag bei 20 %. Die innere Oberfläche des Katalysators betrug 140 m² /g. Das in einem Vakuumkristaller gewonnene Xylitol hatte einen Reinheitsgrad von 96,8 %. Der Gehalt an nicht umgesetzter D-Xylose lag bei 1,5 %. Zusätzlich wurden weitere Zuckerfremdbestandteile in einer Menge von 1,7 % festgestellt, so daß das so erhaltene Xylitol in dieser Herstellungsform ohne ökologisch aufwendige Reinigungsprozeduren nicht als Zuckeraustauschstoff eingesetzt werden konnte. Durch eine Erhöhung der Reaktionstemperatur von 110 auf 125°C konnte zwar der Anteil an nicht umgesetzter D-Xylose auf einen Wert von 1,2 % gesenkt werden, gleichzeitig aber stieg der Anteil an Verunreinigungen organischer Natur auf einen Wert von 3,2 % - darunter Lyxitol und Arabinitol in einer Menge von insgesamt 1,4 % - an. Außerdem war das Reaktionsprodukt mit 36 ppm Ni verunreinigt.

### Beispiel 6 (Vergleichsbeispiel)

Durch ein Hochdruckrohr wie in Beispiel 1 wurde bei einer Temperatur von 115°C und einem Wasserstoffdruck von 300 bar in gleicher Weise wie in Beispiel 1 stündlich eine ebenso große Menge einer 40 %igen wäßrigen Lösung von D-Xylose hydriert, die einen pH-Wert von 6,5 aufwies. Der Katalysator war durch Auftrag von wäßrigen Nickel-und Eisensalz-Lösungen auf einen inerten, kugelförmigen Al₂O₃-Träger (Kugeldurchmesser: 5 mm) und nachfolgende Überführung des Nickels und Eisens in den metallischen Zustand durch Reduzierung im Wasserstoffstrom hergestellt worden. Der Nickelgehalt des Katalysators lag bei 16 %, der Eisengehalt bei 4 %. Die innere Oberfläche des Katalysators lag bei 155 m² /g.

Das durch Eindampfen in einem Vakuumkristaller gewonnene Xylitol hatte einen Reinheitsgrad von 94,6 %. Der Gehalt an nicht umgesetzter D-Xylose lag bei 1,4 %. Zusätzlich wurden organische Verunreinigungen in einer Menge von 4,0 % sowie Ni-Verunreinigungen von 32 ppm und Fe-Verunreinigungen von 11 ppm festgestellt, so daß der erhaltene Zuckeralkohol nicht ohne ökologisch aufwendige Reinigungsmaßnahmen als Zuckeraustauschstoff eingesetzt werden konnte. Schon nach einer Laufzeit von 750 Stunden wurde ein merkliches Nachlassen der Katalysatoraktivität festgestellt.

### Beispiel 7

Ein Hochdruckrohr wie in Beispiel 1 wurde mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m² /g aufwies. Durch dieses Rohr wurden gemeinsam mit der dreifach molaren Menge von unter einem Druck von 300 bar stehendem, hochreinem Wasserstoff stündlich 250 ml einer 40 %igen Lösung von α-D-Glucose in entionisiertem Sauerstofffreiem Trinkwasser mit einem pH-Wert von 7,0 kontinuierlich gepumpt und zwar von unten nach oben aufsteigend.

Wäßrige Lösung und Wasserstoff wurden vorab gemeinsam durch einen Wärmeaustauscher geführt und so hoch erhitzt, daß sie in das Hochdruckrohr mit einer Temperatur von 95°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer α-D-Glucose-Lösung in den Vorwärmer und von da erneut in das Hochdruckrohr gepumpt wurde.

Die farblose und klare wäßrige Lösung wurde entspannt und in einem Fallfilmverdampfer auf einen Zuckeralkoholgehalt von ca. 70 % aufkonzentriert und anschließend nach weiterer Eindampfung in einem Vakuumkristaller unter Kühlung zur Kristallisation gebracht. Es wurde ein weißes, leicht hygroskopisches, geruchloses Festprodukt erhalten, das zu einem feinkristallinen Pulver vermahlen wurde. Das gebildete Sorbitol (D-Glucitol) war ansonsten hochrein und zeigte in der stabilen γ-Form einen Schmelzpunkt von 101°C. Der Gehalt an nicht hydrierter α-D-Glucose lag bei ≦0,1 %. Der Ni-Gehalt lag bei <1 ppm. Der Katalysator war auch nach einer Laufzeit von 9800 Stunden unverändert wirksam.

### Beispiel 8

Durch ein Hochdruckrohr wie in Beispiel 1 wurde bei einer Temperatur von 105°C und einem Wasserstoffdruck von 150 bar der Wasserstoff im umgekehrten Reaktionsfluß, wie in Beispiel 1 beschrieben, der aufsteigenden Lösung von α-D-Glucose entgegengeführt, wobei stündlich eine gleichgroße Menge 40 %iger wäßriger Lösung von α-D-Glucose wie im Beispiel 1 hydriert wurde, die einen pH-Wert von 6,5 aufwies. Der Katalysator war durch Tablettierung von Nickelpulver hergestellt worden. Die Tabletten hatten bei einer Zylinderhöhe von 5 mm und einem Durchmesser von 5 mm eine Druckfestigkeit von 149 N auf die Zylindermantelfläche und eine innere Oberfläche von 62 m² /g.

Nach einer Laufzeit von 4200 Stunden mit unverminderter Wirksamkeit lag der Umsatz der α-D-Glucose bei ≧99,9 %. Der Gehalt an nicht hydrierter α-β-D-Glucose im auskristallisierten Sorbitol (D-Glucitol), das einen Reinheitsgrad von ≧99,5 % aufwies, betrug ≦0,1 %. Der Ni-Gehalt lag bei <1 ppm.

### Beispiel 9

In einem Hochdruckrohr wie in Beispiel 1 wurde bei einer Temperatur von 115°C und einem Wasserstoffdruck von 300 bar in gleicher Weise wie in Beispiel 1 stündlich eine gleichgroße Menge einer 40 %igen wäßrigen Lösung von α-D-Glucose hydriert, die einen pH-Wert von 7,5 aufwies. Der Katalysator war durch Tablettierung einer pulverisierten Nickel-Eisenlegierung gewonnen worden. Die Legierung enthielt einen Eisenanteil in Nickel von 15 %. Die Tabletten hatten bei einer Zylinderhöhe von 5 mm und einem Durchmesser von 5 mm eine Druckfestigkeit von 137 N auf die Zylindermantelfläche und eine innere Oberfläche von 83 m² /g. Das in einem Vakuumkristaller gewonnene kristalline Sorbitol (D-Glucitol) hatte einen Reinheitsgrad von ≧99,5 %. Der Gehalt an nicht umgesetzter α-D-Glucose lag bei 0,1 %. Ni- und Fe-Gehalt lagen zusammen bei <1 ppm. Der Katalysator war nach einer Laufzeit von 8400 Stunden noch unverändert wirksam.

### Beispiel 10

In einem Hochdruckrohr wie in Beispiel 1 wurde bei einer Temperatur von 110°C und einem Wasserstoffdruck von 300 bar in gleicher Weise wie in Beispiel 1 150 ml einer 35 %igen wäßrigen Lösung von α-D-Glucose hydriert, die einen pH-Wert von 6,5 aufwies. Der Katalysator war durch Tablettierung von Kobaltpulver gewonnen worden. Die Tabletten hatten bei einer Zylinderhöhe von 5 mm und einem Durchmesser von 5 mm eine Druckfestigkeit von 225 N auf die Zylindermantelfläche und eine innere Oberfläche von 19 m² /g. Das in einem Vakuum-Drehrohr gewonnene Sorbitol (D-Glucitol) zeigte einen Gehalt an nicht umgesetzter α-D-Glucose von ≦0,2 %. Der Co-Gehalt lag bei <1 ppm. Der Katalysator war nach einer Laufzeit von 1000 Stunden noch unvermindert wirksam.

### Beispiel 11 (Vergleichsbeispiel)

Durch ein Hochdruckrohr wie in Beispiel 1 wurde bei einer Temperatur von 110°C und einem Wasserstoffdruck von 300 bar in gleicher Weise wie in Beispiel 1 in der gleichen Zeit eine ebenso große Menge einer 40 %igen wäßrigen Lösung von α-D-Glucose hydriert, die einen pH-Wert von 6,5 aufwies. Der Katalysator war durch Auftrag einer wäßrigen Nickelsalz-Lösung auf einem inerten kugelförmigen Al₂O₃-Träger (Kugeldurchmesser: 5 mm) und nachfolgende Überführung des Nickels in den metallischen Zustand durch Reduzierung im Wasserstoffstrom hergestellt worden. Der Nickelgehalt des Katalysators lag bei 20 %. Die innere Oberfläche des Katalysators betrug 140 m² /g. Das in einem Vakuumkristaller gewonnene Sorbitol (D-Glucitol) hatte einen Reinheitsgrad von 94,9 %. Der Gehalt an nicht umgesetzter α-D-Glucose lag bei 1,9 %. Zusätzlich wurden weitere Zuckerfremdbestandteile in einer Menge von 3,2 % - darunter Mannitol in einer Menge von 2,1 % - festgestellt, so daß das so erhaltene Sorbitol in dieser Herstellungsform ohne ökologisch aufwendige Reinigungsprozeduren nicht als Zuckeraustauschstoff eingesetzt werden konnte. Durch eine Erhöhung der Reaktionstemperatur von 110 auf 125°C konnte zwar der Anteil an nicht umgesetzter α-D-Glucose auf einen Wert von 0,6 % gesenkt werden, gleichzeitig aber stieg der Anteil an Verunreinigungen organischer Natur auf einen Wert von 6,2 % - darunter Mannitol in einer Menge von 3,9 % - an. Außerdem war das Reaktionsprodukt mit 42 ppm Ni verunreinigt.

### Beispiel 12 (Vergleichsbeispiel)

Durch ein Hochdruckrohr wie in Beispiel 1 wurde bei einer Temperatur von 115°C und einem Wasserstoffdruck von 300 bar in gleicher Weise wie in Beispiel 1 stündlich eine ebenso große Menge einer 40 %igen wäßrigen Lösung von α-D-Glucose hydriert, die einen pH-Wert von 6,5 aufwies. Der Katalysator war durch Auftrag von wäßrigen Nickel-und Eisensalz-Lösungen auf einen inerten, kugelförmigen Al₂O₃-Träger (Kugeldurchmesser: 5 mm) und nachfolgende Überführung des Nickels und Eisens in den metallischen Zustand durch Reduzierung im Wasserstoffstrom hergestellt worden. Der Nickelgehalt des Katalysators lag bei 16 %, der Eisengehalt bei 4 %. Die innere Oberfläche des Katalysators lag bei 155 m² /g.

Das durch Eindampfen in einem Vakuumkristaller gewonnene Sorbitol (D-Glucitol) hatte einen Reinheitsgrad von 93,8 %. Der Gehalt an nicht umgesetzter α-D-Glucose lag bei 1,6 %. Zusätzlich wurden organische Verunreinigungen in einer Menge von 4,6 % - darunter Mannitol in einer Menge von 2,9 % - sowie Ni-Verunreinigungen von 38 ppm und Fe-Verunreinigungen von 16 ppm festgestellt, so daß der erhaltene Zuckeralkohol nicht ohne ökologisch aufwendige Reinigungsmaßnahmen als Zuckeraustauschstoff eingesetzt werden konnte. Schon nach einer Laufzeit von 800 Stunden wurde ein merkliches Nachlassen der Katalysatoraktivität festgestellt.

### Beispiel 13

Ein Hochdruckrohr wie in Beispiel 1 wurde mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m² /g aufwies. Durch dieses Rohr wurden gemeinsam mit der dreifach molaren Menge von unter einem Druck von 300 bar stehendem, hochreinem Wasserstoff stündlich 250 ml einer 40 %igen Lösung von 4-O-β-D-Galactopyranosyl-α-D-glucopyranose in entionisiertem Sauerstoff-freiem Trinkwasser mit einem pH-Wert von 7,0 kontinuierlich gepumpt und zwar von unten nach oben aufsteigend.

Wäßrige Lösung und Wasserstoff wurden vorab gemeinsam durch einen Wärmeaustauscher geführt und so hoch erhitzt, daß sie in das Hochdruckrohr mit einer Temperatur von 90°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer 4-O-β-D-Galactopyranosyl-α-D-glucopyranose in den Vorwärmer und von da erneut in das Hochdruckrohr gepumpt wurde.

Die farblose und klare wäßrige Lösung wurde entspannt und in einem Fallfilmverdampfer auf einen Zuckeralkoholgehalt von ca. 80 % aufkonzentriert und anschließend nach weiterer Eindampfung in einem Vakuumkristaller unter Kühlung zur Kristallisation gebracht. In Abhängigkeit von den Kristallisationsumständen und dem Restwassergehalt der eingedampften Lösung ließ sich dabei entweder das Dihydrat mit einem Schmelzpunkt von 76-78°C oder das Monohydrat mit einem Schmelzpunkt von 121-123°C isolieren. Das gebildete 4-O-β-D-Galactopyranosyl-D-glucitol war ansonsten hochrein (Reinheitsgrad ≧99,6 %). Der Gehalt an nicht hydrierter 4-O-β-D-Galactopyranosyl-α-D-glucopyranose lag bei ≦0,1 %. Der Gehalt an Sorbitol bzw. Dulcitol betrug ≦0,1 %. 4-O-β-D-Galactopyranosyl-D-mannitol bzw. Mannitol konnten nicht nachgewiesen werden. Der Ni-Gehalt lag bei <1 ppm. Der Katalysator war auch nach einer Laufzeit von 8700 Stunden unverändert wirksam.

### Beispiel 14

Durch ein Hochdruckrohr, wie in Beispiel 1 verwendet, wurde bei einer Temperatur von 105°C und einem Wasserstoffdruck von 150 bar der Wasserstoff im umgekehrten Reaktionsfluß, wie in Beispiel 1 beschrieben, der aufsteigenden Lösung von 4-O-β-D-Galactopyranosyl-α-D-glucopyranose entgegengeführt, wobei stündlich eine gleichgroße Menge 40 %iger wäßriger Lösung von 4-O-β-D-Galactopyranose-α-D-glucopyranose wie im Beispiel 1 hydriert wurde, die einen pH-Wert von 6,5 aufwies. Der Katalysator wurde durch Tablettierung von Nickelpulver hergestellt. Die Tabletten hatten bei einer Zylinderhöhe von 5 mm und einem Durchmesser von 5 mm eine Druckfestigkeit von 149 N auf die Zylindermantelfläche und eine innere Oberfläche von 62 m² /g.

Nach einer Laufzeit von 2500 Stunden mit unverminderter Wirksamkeit lag der Gehalt an 4-O-β-D-Galactopyranosyl-D-glucitol des in einem Rotationsverdampfer zur Trockne eingedampften Reaktionsgemisches bei 99,4 %. Der Gehalt an nicht hydrierter 4-O-β-D-Galactopyranosyl-α-D-glucopyranose betrug ≦0,1 %. Der Gehalt an Sorbitol bzw. Dulcitol lag bei 0,1 %. 4-O-β-D-Galactopyranosyl-D-mannitol bzw. Mannitol konnten nicht nachgewiesen werden. Der Ni-Gehalt lag bei <1 ppm.

### Beispiel 15

In einem Hochdruckrohr, wie in Beispiel 1 verwendet, wurde bei einer Temperatur von 115°C und einem Wasserstoffdruck von 300 bar in gleicher Weise wie in Beispiel 1 stündliche eine gleichgroße Menge einer 40 %igen wäßrigen Lösung von 4-O-β-D-Galactopyranosyl-α-D-glucopyranose hydriert, die einen pH-Wert von 7,5 aufwies. Der Katalysator wurde durch Tablettierung einer pulverisierten Nickel-Eisenlegierung gewonnen. Die Legierung enthielt einen Eisenanteil in Nickel von 15 %. Die Tabletten hatten bei einer Zylinderhöhe von 5 mm und einem Durchmesser von 5 mm eine Druckfestigkeit von 137 N auf die Zylindermantelfläche und eine innere Oberfläche von 83 m² /g. Das in einem Vakuumkristaller gewonnene 4-O-β-D-Galactopyranosyl-α-D-glucitol hatte einen Reinheitsgrad von 99,3 %. Der Gehalt an nicht umgesetzter 4-O-β-D-Galactopyranosyl-α-D-glucose lag bei 0,1 %. Der Sorbitol- bzw. Dulcitolgehalt betrug 0,1 %. 4-O-β-D-Galactopyranosyl-D-mannitol bzw. Mannitol konnte nicht nachgewiesen werden. Ni- und Fe-Gehalt lagen bei <1 ppm. Der Katalysator war nach einer Laufzeit von 8000 Stunden noch unverändert wirksam.

### Beispiel 16

In einem Hochdruckrohr, wie in Beispiel 1 verwendet, wurde bei einer Temperatur von 110°C und einem Wasserstoffdruck von 200 bar in gleicher Weise wie in Beispiel 1 eine ebenso große Menge einer 30 %igen wäßrigen Lösung von 4-O-β-D-Galactopyranosyl-α-D-glucopyranose hydriert, die einen pH-Wert von 6,5 aufwies. Der Katalysator wurde durch Tablettierung einer pulverisierten Nickel-Kobaltlegierung gewonnen. Die Legierung enthielt einen Kobaltanteil in Nickel von 10 %. Die Tabletten hatten bei einer Zylinderhöhe von 5 mm und einem Durchmesser von 5 mm eine Druckfestigkeit von 147 N auf die Zylindermantelfläche und eine innere Oberfläche von 29 m² /g. Das in einem Vakuum-Drehrohr gewonnene 4-O-β-D-Galactopyranosyl-α-D-glucitol hatte einen Reinheitsgrad von 99,1 %. Der Gehalt an nicht umgesetzter 4-O-β-D-Galactopyranosyl-α-D-glucopyranose lag bei 0,2 %. Der Sorbitolgehalt betrug 0,2 %. Der Dulcitolanteil betrug 0,15 %. 4-O-β-D-Galactopyranosyl-D-mannitol konnte nicht nachgewiesen werden. Ni- und Co-Gehalt lagen bei <1 ppm. Der Katalysator war nach einer Laufzeit von 1000 Stunden noch unvermindert wirksam.

### Beispiel 17 (Vergleichsbeispiel)

Durch ein Hochdruckrohr, wie in Beispiel 1 verwendet, wurde bei einer Temperatur von 100°C und einem Wasserstoffdruck von 300 bar in gleicher Weise wie in Beispiel 1 in der gleichen Zeit eine ebenso große Menge einer 40 %igen wäßrigen Lösung von 4-O-β-D-Galactopyranosyl-α-D-glucopyranose hydriert, die einen pH-Wert von 7,0 aufwies. Der Katalysator war durch Auftrag einer wäßrigen Nickelsalz-Lösung auf einem inerten kugelförmigen Al₂O₃-Träger (Kugeldurchmesser: 5 mm) und nachfolgende Überführung des Nickels in den metallischen Zustand durch Reduzierung im Wasserstoffstrom hergestellt worden. Der Nickelgehalt des Katalysators lag bei 20 %. Die innere Oberfläche des Katalysators betrug 145 m² /g. Das in einem Vakuumkristaller gewonnene 4-O-β-D-Galactopyranosyl-α-D-glucitol hatte einen Reinheitsgrad von 93,9 %. Der Gehalt an nicht umgesetzter 4-O-β-D-Galactopyranosyl-α-D-glucopyranose lag bei 1,9 %. Sorbitol- und Dulcitolgehalt lagen bei 0,4 bzw. 0,2 %. Zusätzlich wurden weitere Verunreinigungen in einer Höhe von 3,6 %, darunter 4-O-β-D-Galactopyranosyl-D-mannitol mit 2,6 %, festgestellt, so daß das so erhaltene 4-O-β-D-Galactopyranosyl-α-D-glucitol in dieser Herstellungsform ohne ökologisch aufwendige Reinigungsprozeduren nicht als Zuckeraustauschstoff eingesetzt werden konnte. Ferner wurde schon nach einer Laufzeit von 800 Stunden ein Nachlassen der Katalysatoraktivität beobachtet. Durch eine Erhöhung der Reaktionstemperatur von 100 auf 120°C konnte zwar der Anteil an nicht umgesetzter 4-O-β-D-Galactopyranosyl-α-D-glucopyranose auf einen Wert von 0,6 % gesenkt werden, gleichzeitig aber stieg der Anteil an Verunreinigungen auf einen Wert von 6,4 % an. Darin war 4-O-β-D-Galactopyranosyl-D-mannitol mit 3,2 % enthalten. Außerdem war das Reaktionsprodukt mit 42 ppm Ni verunreinigt.

### Beispiel 18 (Vergleichsbeispiel)

Durch ein Hochdruckrohr, wie in Beispiel 1 verwendet, wurde bei einer Temperatur von 100°C und einem Wasserstoffdruck von 300 bar in gleicher Weise wie in Beispiel 1 stündlich eine ebenso große Menge einer 40 %igen wäßrigen Lösung von 4-O-β-D-Galactopyranosyl-α-D-glucopyranose hydriert, die einen pH-Wert von 6,5 aufwies. Der Katalysator war durch Auftrag von wäßrigen Nickel- und Eisensalz-Lösungen auf einen inerten, kugelförmigen Al₂O₃-Träger (Kugeldurchmesser: 5 mm) und nachfolgende Überführung des Nickels und Eisens in den metallischen Zustand durch Reduzierung im Wasserstoffstrom hergestellt worden. Der Nickelgehalt des Katalysators lag bei 16 %, der Eisengehalt bei 4 %. Die innere Oberfläche des Katalysators lag bei 151 m² /g. Das durch Eindampfen in einem Vakuumkristaller gewonnene 4-O-β-D-Galactopyranosyl-α-D-glucitol hatte einen Reinheitsgrad von 91,2 %. Der Gehalt an nicht umgesetzter 4-O-β-D-Galactopyranosyl-α-D-glucopyranose lag bei 1,6 %. Der Sorbitol- bzw . Dulcitolgehalt lag bei 0,3 bzw. 0,1 %. Zusätzlich wurden weitere organische Verunreinigungen in einer Höhe von 6,8 %, darunter 4-O-β-D-Galactopyranosyl-D-mannitol mit 2,9 %, sowie Ni-Verunreinigungen von 36 ppm und Fe-Verunreinigungen von 14 ppm festgestellt, so daß der erhaltene Zuckeralkohol nicht ohne ökologisch aufwendige Reinigungsmaßnahmen als Zuckeraustauschstoff eingesetzt werden konnte.

### Beispiel 19

Ein Hochdruckrohr wie in Beispiel 1 wurde mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 174 N auf die Zylindermantelfläche und eine innere Oberfläche von 33 m² /g aufwies. Durch dieses Rohr wurden gemeinsam mit der fünffach molaren Menge von unter einem Druck von 300 bar stehendem, hochreinem Wasserstoff stündlich 250 ml einer 40 %igen Lösung von 4-O-α-D-Glucopyranosyl-α-D-glucopyranose in entionisiertem Sauerstoff-freiem Trinkwasser mit einem pH-Wert von 7,0 kontinuierlich gepumpt und zwar von unten nach oben aufsteigend.

Wäßrige Lösung und Wasserstoff wurden vorab gemeinsam durch einen Wärmeaustauscher geführt und so hoch erhitzt, daß sie in das Hochdruckrohr mit einer Temperatur von 80°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer 4-O-α-D-Glucopyranosyl-α-D-glucopyranose in den Vorwärmer und von da erneut in das Hochdruckrohr gepumpt wurde.

Die farblose und klare wäßrige Lösung wurde entspannt und in einem Fallfilmverdampfer auf einen Zuckeralkoholgehalt von ca. 80 % aufkonzentriert und anschließend nach weiterer Eindampfung in einem Vakuumkristaller unter Kühlung und gegebenenfalls Hinzufügung von Impfkristallen zur Kristallisation gebracht. Das kristalline 4-O-α-D-Glucopyranosyl-D-sorbitol zeigte einen Reinheitsgrad von ≧ 99,6 %. Der Gehalt an nicht hydrierter 4-O-α-D-Glucopyranosyl-α-D-glucopyranose lag bei ≦0,1 %. Der Gehalt an Sorbitol betrug ≦0,3 %. Mannitol konnte nicht nachgewiesen werden. Der Ni-Gehalt lag bei <1 ppm. Der Katalysator war auch nach einer Laufzeit von 6200 Stunden unverändert wirksam.

### Beispiel 20

Durch ein Hochdruckrohr, wie in Beispiel 1 verwendet, wurde bei einer Temperatur von 85°C und einem Wasserstoffdruck von 150 bar der Wasserstoff im umgekehrten Reaktionsfluß, wie in Beispiel 1 beschrieben, der aufsteigenden Lösung von 4-O-α-D-Glucopyranosyl-α-D-glucopyranose entgegengeführt, wobei stündlich eine gleichgroße Menge 40 %iger wäßriger Lösung von 4-O-α-D-Glucopyranosyl-α-D-glucopyranose wie im Beispiel 1 hydriert wurde, die einen pH-Wert von 6,5 aufwies. Der Katalysator wurde durch Tablettierung von Nickelpulver hergestellt. Die Tabletten hatten bei einer Zylinderhöhe von 5 mm und einem Durchmesser von 5 mm eine Druckfestigkeit von 149 N auf die Zylindermantelfläche und eine innere Oberfläche von 62 m² /g.

Nach einer Laufzeit von 2500 Stunden mit unverminderter Wirksamkeit lag der Gehalt an 4-O-α-D-Glucopyranosyl-D-sorbitol des in einem Rotationsverdampfer zur Trockne eingedampften Reaktionsgemisches bei 99,4 %. Der Gehalt an nicht hydrierter 4-O-α-D-Glucopyranosyl-α-D-glucopyranose betrug ≦0,1 %. Der Gehalt an Sorbitol lag bei 0,3 %. Der Ni-Gehalt lag bei <1 ppm.

### Beispiel 21

In einem Hochdruckrohr, wie in Beispiel 1 verwendet, wurde bei einer Temperatur von 80°C und einem Wasserstoffdruck von 300 bar in gleicher Weise wie in Beispiel 1 stündlich eine gleich große Menge einer 40 %igen wäßrigen Lösung von 4-O-α-D-Glucopyranosyl-α-D-glucopyranose hydriert, die einen pH-Wert von 7,5 aufwies. Der Katalysator wurde durch Tablettierung einer pulverisierten Nickel-Eisenlegierung gewonnen. Die Legierung enthielt einen Eisenanteil in Nickel von 15 %. Die Tabletten hatten bei einer Zylinderhöhe von 5 mm und einem Durchmesser von 5 mm eine Druckfestigkeit von 137 N auf die Zylindermantelfläche und eine innere Oberfläche von 83 m² /g. Das in einem Vakuumverdampfer gewonnene 4-O-α-D-Glucopyranosyl-α-D-sorbitol hatte einen Reinheitsgrad von 99,3 %. Der Gehalt an nicht umgesetzter 4-O-α-D-Glucopyranosyl-α-D-glucose lag bei 0,1 %. Der Sorbitolgehalt betrug 0,3 %. Ni- und Fe-Gehalt lagen bei <1 ppm. Der Katalysator war nach einer Laufzeit von 4200 Stunden noch unverändert wirksam.

### Beispiel 22

In einem Hochdruckrohr, wie in Beispiel 1 verwendet, wurde bei einer Temperatur von 100°C und einem Wasserstoffdruck von 200 bar in gleicher Weise wie in Beispiel 1 eine ebenso große Menge einer 30 %igen wäßrigen Lösung von 4-O-α-D-Glucopyranosyl-α-D-glucopyranose hydriert, die einen pH-Wert von 6,5 aufwies. Der Katalysator wurde durch Tablettierung von Kobaltpulver gewonnen. Die Tabletten hatten bei einer Zylinderhöhe von 5 mm und einem Durchmesser von 5 mm eine Druckfestigkeit von 225 N auf die Zylindermantelfläche und eine innere Oberfläche von 19 m² /g. Das in einem Vakuum-Drehrohr gewonnene 4-O-α-D-Glucopyranosyl-α-D-sorbitol hatte einen Reinheitsgrad von 99,1 %. Der Gehalt an nicht umgesetzter 4-O-α-D-Glucopyranosyl-α-D-glucopyranose lag bei 0,1 %. Der Sorbitolgehalt betrug 0,2 %. Der Co-Gehalt lag bei <1 ppm. Der Katalysator war nach einer Laufzeit von 1000 Stunden noch unvermindert wirksam.

### Beispiel 23 (Vergleichsbeispiel)

Durch ein Hochdruckrohr, wie in Beispiel 1 verwendet, wurde bei einer Temperatur von 100°C und einem Wasserstoffdruck von 300 bar in gleicher Weise wie in Beispiel 1 in der gleichen Zeit eine ebenso große Menge einer 40 %igen wäßrigen Lösung von 4-O-α-D-Glucopyranosyl-α-D-glucopyranose hydriert, die einen pH-Wert von 7,0 aufwies. Der Katalysator war durch Auftrag einer wäßrigen Nickelsalz-Lösung auf einem inerten kugelförmigen Al₂O₃-Träger (Kugeldurchmesser: 5 mm) und nachfolgende Überführung des Nickels in den metallischen Zustand durch Reduzierung im Wasserstoffstrom hergestellt worden. Der Nickelgehalt des Katalysators lag bei 20 %. Die innere Oberfläche des Katalysators betrug 148 m²/g. Das in einem Vakuumverdampfer gewonnene 4-O-α-D-Glucopyranosyl-α-D-sorbitol hatte einen Reinheitsgrad von 92,9 %. Der Gehalt an nicht umgesetzter 4-O-α-D-Glucopyranosyl-α-D-glucopyranose lag bei 1,4 %. Der Sorbitolgehalt lag bei 3,6 %. Zusätzlich wurden unbekannte Verunreinigungen in einer Höhe von 2,1 % festgestellt, so daß das so erhaltene 4-O-α-D-Glucopyranosyl-α-D-sorbitol in dieser Herstellungsform ohne ökologisch aufwendige Reinigungsprozeduren nicht als Zuckeraustauschstoff eingesetzt werden konnte. Ferner wurde schon nach einer Laufzeit von 700 Stunden ein Nachlassen der Katalysatoraktivität beobachtet. Durch eine Erhöhung der Reaktionstemperatur von 100 auf 120°C konnte zwar der Anteil an nicht umgesetzter 4-O-α-D-Glucopyranosyl-α-D-glucopyranose auf einen Wert von 0,2 % gesenkt werden, gleichzeitig aber stieg der Anteil an Sorbitol auf einen Wert von 4,2 % an. Außerdem war das Reaktionsprodukt mit 48 ppm Ni verunreinigt.

### Beispiel 24 (Vergleichsbeispiel)

Durch ein Hochdruckrohr, wie in Beispiel 1 verwendet, wurde bei einer Temperatur von 100°C und einem Wasserstoffdruck von 300 bar in gleicher Weise wie in Beispiel 1 stündlich eine ebenso große Menge einer 40 %igen wäßrigen Lösung von 4-O-α-D-Glucopyranosyl-α-D-glucopyranose hydriert, die einen pH-Wert von 6,5 aufwies. Der Katalysator war durch Auftrag von wäßrigen Nickel- und Eisensalz-Lösungen auf einen inerten, kugelförmigen Al₂O₃-Träger (Kugeldurchmesser: 5 mm) und nachfolgende Überführung des Nickels und Eisens in den metallischen Zustand durch Reduzierung im Wasserstoffstrom hergestellt worden. Der Nickelgehalt des Katalysators lag bei 16 %, der Eisengehalt bei 4 %. Die innere Oberfläche des Katalysators lag bei 151 m² /g. Das durch Eindampfen in einem Rotationsverdampfer gewonnene 4-O-α-D-Glucopyranosyl-α-D-sorbitol hatte einen Reinheitsgrad von 93,4 %. Der Gehalt an nicht umgesetzter 4-O-α-D-Glucopyranosyl-α-D-glucopyranose lag bei 1,0 %. Der Sorbitolgehalt lag bei 4,3 %. Zusätzlich wurden unbekannte organische Verunreinigungen in einer Höhe von 1,3 % sowie Ni-Verunreinigungen von 31 ppm und Fe-Verunreinigungen von 12 ppm festgestellt, so daß der erhaltene Zuckeralkohol nicht ohne ökologisch aufwendige Reinigungsmaßnahmen als Zuckeraustauschstoff eingesetzt werden konnte.

## Patentansprüche

1. Verfahren zur Herstellung von epimerenfreien Zuckeralkoholen aus der Gruppe von Xylitol, Sorbitol (D-Glucitol), 4-O-β-D-Galactopyranosyl-D-glucitol und 4-O-α-D-Glucopyranosyl-D-sorbitol durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 500 bar und Temperaturen von 60 bis 125°C im Festbettverfahren in einer Reaktionszone über als Hydrierungskatalysatoren dienenden trägerfreien Formkörpern mit einer Druckfestigkeit von mehr als 50 N, vorzugsweise 100 bis 400 N, auf die Formkörperoberfläche und einer inneren Oberfläche von 10 bis 90 m² /g von einem oder mehreren Elementen der Eisengruppe des Periodensystems durchführt.

2. Verfahren nach Anspruch 1, dadurch gekennzeichnet, daß es sich bei den Formkörpern um solche aus verpreßten Metallpulvern aus Nickel, Kobalt oder ihren Gemischen oder Legierungen untereinander oder mit Eisen hergestellte mit makroskopisch glatter Oberfläche handelt.

3. Verfahren nach Anspruch 1, dadurch gekennzeichnet, daß die Hydrierungskatalysatoren zylinderförmige oder kugelförmige Formkörper mit einem Durchmesser von 3-7 mm sind.

4. Verfahren nach Anspruch 1, dadurch gekennzeichnet, daß man die Hydrierung der Zucker in 15 bis 45 %iger wäßriger Lösung bei einem pH-Wert von 3,5 bis 10,5 durchführt.

## Claims

1. Process for the preparation of epimer-free sugar alcohols from the group consisting of xylitol, sorbitol (D-glucitol), 4-O-β-D-galactopyranosyl-D-glucitol and 4-O-α-D-glucopyranosyl-D-sorbitol by catalytic hydrogenation of the corresponding sugars D-xylose, α-D-glucose, 4-O-β-D-galactopyranosyl-α-D-glucopyranose or 4-O-α-D-glucopyranosyl-α-D-glucopyranose in aqueous solution using hydrogen under elevated pressure and at elevated temperature, characterized in that the hydrogenation is carried out continuously at a hydrogen pressure of 100 to 500 bar and temperatures of 60 to 125°C in a reaction zone by the fixed bed process over hydrogenation catalysts in the form of carrier-free mouldings having a compressive strength of more than 50 N, preferably 100 to 400 N, onto the surface of the mouldings, and an internal surface of 10 to 90 m²/g, these mouldings being made from one or more elements of the iron group of the periodic table.

2. Process according to Claim 1, characterized in that the mouldings are of the type prepared from pressed metal powders composed of nickel, cobalt or mixtures thereof or alloys with one another or with iron, having a macroscopically smooth surface.

3. Process according to Claim 1, characterized in that the hydrogenation catalysts are cylindrical or spherical mouldings having a diameter of 3-7 mm.

4. Process according to Claim 1, characterized in that the hydrogenation of the sugars is carried out in 15 to 45% strength aqueous solution at a pH of 3.5 to 10.5.

## Revendications

1. Procédé pour fabriquer des alcools de sucre ne contenant pas d'épimères, appartenant au groupe comportant le xylitol, le sorbitol (D-glucitol), le 4-O-β-D-galactopyranosyl-D-glucitol et le 4-O-α-D-glucopyranosyl-D-sorbitol, par hydrogénation catalytique des sucres correspondants D-xylose, α-D-glucose, 4-O-β-D-galactopyranosyl-α-D-glucopyranose ou 4-O-α-D-glucopyranosyl-α-D-glucopyranose en solution aqueuse avec de l'hydrogène sous pression élevée et à température élevée, caractérisé en ce que l'on exécute l'hydrogénation de manière continue sous une pression d'hydrogène de 100 à 500 bar et à des températures de 60 à 125°C dans un procédé à lit fixe dans une zone de réaction sur des corps moulés sans support servant de catalyseurs d'hydrogénation, présentant une résistance à la compression supérieure à 50 N, de préférence de 100 à 400 N sur la surface des corps moulés, et une surface intérieure de 10 à 90 m²/g d'un ou plusieurs éléments du groupe du fer du système périodique.

2. Procédé selon la revendication 1,
caractérisé en ce que les corps moulés sont fabriqués à partir de poudres métalliques comprimées de nickel, de cobalt ou de leurs mélanges ou de leurs alliages entre eux ou avec du fer et présentent une surface lisse en vue macroscopique.

3. Procédé selon la revendication 1,
caractérisé en ce que les catalyseurs d'hydrogénation sont des corps moulés de forme cylindrique ou sphérique avec un diamètre de 3 à 7 mm.

4. Procédé selon la revendication 1,
caractérisé en ce que l'on exécute l'hydrogénation des sucres dans une solution aqueuse à 15 à 45% à un pH de 3,5 à 10,5.
